# EUROPEAN PATENT APPLICATION

(11) **EP 2 146 207 A1**
(43) Date of publication of application: **20.01.2010**
(21) Application number: 08851516.8
(22) Date of filing: 20.11.2008
(51) Int. Cl.: G01N 33/543, G01N 33/545

(54) **MEASUREMENT REAGENT, IMMUNE NEPHELOMETRY USING THE SAME, AND ANALYTE ANALYSIS TOOL**

(30) Priority: 21.11.2007 JP 2007302182
(71) Applicant: Arkray, Inc., Kyoto-shi, Kyoto 601-8045 (JP)
(72) Inventor: TAKAGI, Hidenori, Kyoto-shi, Kyoto 601-8045 (JP)
(74) Representative: Behnisch, Werner
(86) International application number: PCT/JP2008/071121
(87) International publication number: WO 2009/066731

(57) **Abstract**

A measuring reagent is provided that allows the amounts of insoluble carrier particles and monoclonal antibodies to be reduced, can improve measurement sensitivity with respect to an object to be measured in a low concentration range, allows the object to be measured in a wide concentration range, and is used for a turbidimetric immunoassay applicable to a sample analysis tool such as a test piece. The measuring reagent includes two types of insoluble carrier particle groups that are different in mean particle size from each other. The insoluble carrier particles contained in one insoluble carrier particle group support polyclonal antibodies and monoclonal antibodies, and the insoluble carrier particles contained in the other insoluble carrier particle group support at least one of the polyclonal antibodies and the monoclonal antibodies. Preferably, the former is an insoluble carrier particle group with a small mean particle size and the latter is an insoluble carrier particle group with a large mean particle size.

## Description

### Technical Field

The present invention relates to measuring reagents, as well as turbidimetric immunoassays and sample analysis tools using the same.

### Background Art

Conventionally, for example, an enzyme immunoassay method (EIA method) and a turbidimetric immunoassay are used as immunological measurement methods in laboratory tests. Particularly, a turbidimetric immunoassay in which latex particles are used as insoluble carrier particles is used widely recently since it has an improved measurement sensitivity (see, for instance, Patent Documents 1 to 4) and allows an autoanalyzer to be used.

The turbidimetric immunoassay using latex particles also is referred to as a latex agglutination turbidimetric immunoassay. This method is a measurement method that uses latex particles to supporting antibodies to an object to be measured, and utilizes the fact that the supported antibodies and the object to be measured (antigens) contained in a specimen undergo an antigen-antibody reaction and thereby the latex particles agglutinate. Examples of the object to be measured contained in the specimen include one that exists in blood in a wide concentration range, such as C-reactive protein (CRP) that appears in blood when, for example, an inflammatory response occurs in the body. Therefore, the turbidimetric immunoassay using latex particles is required not only to have an improved sensitivity described above but also to make it possible to determine the quantity of the object to be measured in a wide concentration range. In this connection, methods using two types of latex particles have been proposed as described in, for example, the following Patent Documents 1 to 4.

Patent Document 1 discloses a method of using two types of latex particles that contain different amounts of identical antigens or identical antibodies added thereto and that are different in mean particle size from each other. Furthermore, Patent Document 2 discloses a method in which two or more types of latex particles are mixed that have been sensitized by antibodies or antigens and that are different in mean particle size from each other, which then is irradiated with light of a specific wavelength. Patent Document 3 discloses a method using two types of latex particles that are different in mean particle size from each other, with monoclonal antibodies having been immobilized thereto. Furthermore, Patent Document 4 discloses a method using a mixture obtained by mixing, in a specific ratio, two types of antibody-sensitized latex particles that are sensitized by different monoclonal antibodies, respectively.
[Nonpatent Document 1] JP 63(1988)-14783 B
[Nonpatent Document 2] JP 2588174 B
[Nonpatent Document 3] JP 2005-106609A
[Nonpatent Document 4] JP 3598701 B

### Disclosure of Invention

However, the turbidimetric immunoassay using two types of latex particles that are different in mean particle size from each other as disclosed in Patent Documents 1 to 3 is not suited to provide, for example, a concentration range and measurement sensitivity that make it possible to measure an object to be measured (antigens) as follows. That is, in the turbidimetric immunoassay, for example, in order to obtain measurement sensitivity for a low concentration of an object to be measured, it is necessary to increase the antibody sensitizing dose of particles with a large mean particle size (large particles) and to reduce the antibody sensitizing dose of particles with a small mean particle size (small particles). However, when the antibody sensitizing dose to the small particles is reduced, it is necessary to increase the amount of small particles in the antigen-antibody reaction in order to obtain a concentration range in which the object to be measured can be measured. As a result, the cost required for latex particles increases. Furthermore, the total particle concentration increases in the antigen-antibody reaction and therefore nonspecific agglutination tends to occur. Moreover, when the amount of the small particles is increased excessively in the antigen-antibody reaction, the measurement sensitivity to a low concentration of the object to be measured is deteriorated adversely.
On the other hand, when instead of increasing the amount of small particles in the antigen-antibody reaction, the amount of antibodies that are allowed to sensitize small particles in the sensitization reaction is increased, in the case of a low concentration of an object to be measured, a larger amount of small particles react with the object to be measured before large particles react therewith. Thus, it is difficult to obtain measurement sensitivity to the low concentration of the object to be measured. Furthermore, when the amount of the small particles is reduced, a prozone phenomenon may occur in some cases, and the number of particles to agglutinate is reduced. Thus, it is difficult to obtain the sensitivity in the range of high concentrations. As described above, in the turbidimetric immunoassay using two types of latex particles that are different in mean particle size from each other, it is difficult to control by, for example, a combination of optimum mean particle sizes, the ratio of antibodies to be allowed to sensitize latex particles, the mixing ratio of two types of latex particles, or the concentration of latex particles in the antigen-antibody reaction. Thus, it is difficult to obtain the measurement sensitivity to a low concentration of an object to be measured and the concentration range in which an object to be measured can be measured.

On the other hand, as disclosed in Patent Documents 3 and 4 described above, the method using latex particles that support monoclonal antibodies is intended to prevent the aforementioned nonspecific agglutination from occurring and obtain the measurement sensitivity. However, this method uses monoclonal antibodies and thus the cost increases, which is a problem. Furthermore, since the monoclonal antibodies each have one antigen recognition site, the use of only one type of antibody does not allow agglutination to occur through the antigen-antibody reaction except for the case where the antigens of the object to be measured are multivalents or multimers. Accordingly, when monoclonal antibodies are used, it is necessary to combine a plurality of them and it is necessary to select antibodies that satisfy such conditions that, for example, they are different in antigen recognition site from each other, are not contiguous, and are not subjected to steric hindrance. Therefore it is complicated. Moreover, generally, the use of monoclonal antibodies alone results in low measurement sensitivity to a low concentration of an object to be measured. Furthermore, in latex agglutination turbidimetric immunoassay, when the concentration of the object to be measured (antigens) in a specimen is high, for example, a low dilution rate of the specimen results in still a high concentration of the object to be measured in the diluted specimen (measurement sample), and therefore there is a problem in that the measurement range is narrow. Accordingly, the latex agglutination turbidimetric immunoassay is difficult to apply to a measurement method in which the specimen dilution rate is low, such as a measurement method using a microchip, for example.

Therefore, the present invention is intended to provide a measuring reagent that, for example, allows the amounts of insoluble carrier particles and monoclonal antibodies to be reduced, can improve measurement sensitivity with respect to an object to be measured (antigens) in a low concentration range, allows the object to be measured in a wide concentration range, and also is applicable to a sample analysis tool such as a test piece, as well as a turbidimetric immunoassay and a sample analysis tool using the same.

In order to achieve the aforementioned object, a measuring reagent of the present invention is a measuring reagent that is used for a turbidimetric immunoassay,
wherein the measuring reagent includes an insoluble carrier particle group containing a plurality of insoluble carrier particles that support antibodies,
the insoluble carrier particle group includes two or more types of insoluble carrier particle groups that are different in mean particle size from each other, and
in the two or more types of insoluble carrier particle groups that are different in mean particle size from each other, insoluble carrier particles contained in at least one type of insoluble carrier particle group support polyclonal antibodies and monoclonal antibodies, and
insoluble carrier particles contained in another type of insoluble carrier particle group support at least one of the polyclonal antibodies and the monoclonal antibodies.

A turbidimetric immunoassay of the present invention is a turbidimetric immunoassay in which, using a measuring reagent including an insoluble carrier particle group containing a plurality of insoluble carrier particles that support antibodies, and
an object to be measured to serve as antigens and the antibodies are reacted with each other, so that the insoluble carrier particle group is agglutinated,
wherein the measuring reagent to be used is a measuring reagent of the present invention.

A test analysis tool of the present invention is a sample analysis tool that includes a substrate and a measuring reagent and that is used for a turbidimetric immunoassay, with the measuring reagent being disposed on the substrate,
wherein the measuring reagent is a measuring reagent according to the present invention.

In order to achieve the above-mentioned object, the present inventors made a series of studies. As a result, they found that the use of a measuring reagent including an insoluble carrier particle group allowed an object to be measured (antigens) in a range from a low concentration to a high concentration to be measured with small amounts of particles and monoclonal antibodies, and thereby completed the present invention. In the measuring reagent, the insoluble carrier particle group includes two or more types of insoluble carrier particle groups that are different in mean particle size from each other, polyclonal antibodies and monoclonal antibodies are supported by at least one type of insoluble carrier particle group, and at least one of the aforementioned antibodies is supported by another insoluble carrier particle group. According to the present invention, the quantity of an object to be measured in a wide concentration range can be determined and an object to be measured with a low concentration can be measured with high sensitivity. Furthermore, the present invention allows, for example, a sample analysis tool to be used and makes it possible to reduce the cost.

### Brief Description of Drawings

FIG. 1 is a graph showing the results of measurements of the relationship between the CRP concentration and the variation in absorbance using various measuring reagents in examples of the present invention.
FIG. 2 is a graph showing the results of measurements of the relationship between the CRP concentration and the variation in absorbance using various measuring reagents in other examples of the present invention.
FIG. 3 shows an example of a sample analysis tool of the present invention; 3(A) is a plan view of the sample analysis tool, and 3(B) is a cross-sectional view that is viewed from the I-I direction of the sample analysis tool indicated in 3(A).
FIG. 4 is a graph showing the results of measurements of the relationship between the CRP concentration and the variation in absorbance using a sample analysis tool with a dry-type measuring reagent disposed therein in another example of the present invention.

### Best Mode for Currying Out the Invention

In the measuring reagent of the present invention, it is preferable that the insoluble carrier particle group include two types of insoluble carrier particle groups that are different in mean particle size from each other, and
in the two types of insoluble carrier particle groups, insoluble carrier particles of an insoluble carrier particle group with a small mean particle size support (immobilize) polyclonal antibodies and monoclonal antibodies, and
insoluble carrier particles of an insoluble carrier particle group with a large mean particle size support (immobilize) at least one of the polyclonal antibodies and the monoclonal antibodies. The measuring reagent of the present invention is not limited to the above-mentioned mode and can be, for example, the following mode.

That is, it may be a mode where in the measuring reagent of the present invention, the insoluble carrier particle group includes two types of insoluble carrier particle groups that are different in mean particle size from each other, and
in the two types of insoluble carrier particle groups, insoluble carrier particles of an insoluble carrier particle group with a large mean particle size support polyclonal antibodies and monoclonal antibodies, and
insoluble carrier particles of an insoluble carrier particle group with a small mean particle size support at least one of the polyclonal antibodies and the monoclonal antibodies.

In the measuring reagent of the present invention, the insoluble carrier particles may be latex particles.

In the measuring reagent of the present invention, it is preferable that the antibody concentration ratio between the polyclonal antibodies and the monoclonal antibodies that are allowed to sensitize the insoluble carrier particles of the insoluble carrier particle group with the small mean particle size be 1:9 to 7:3.

In the sample analysis tool of the present invention, it is preferable that the antibody concentration ratio between the polyclonal antibodies and the monoclonal antibodies that are allowed to sensitize the insoluble carrier particles of the insoluble carrier particle group with the small mean particle size be 1:9 to 7:3. The sample analysis tool of the present invention may be any one of, for example, a test piece, a cartridge, and a microchip.

Hereinafter, the present invention is described in detail but is not limited thereto.

### <Turbidimetric Immunoassay>

As described above, the turbidimetric immunoassay of the present invention is a turbidimetric immunoassay in which, using a measuring reagent including an insoluble carrier particle group containing a plurality of insoluble carrier particles that support antibodies, an object to be measured to serve as antigens and the antibodies are reacted with each other, so that the insoluble carrier particle group is agglutinated, wherein the measuring reagent to be used is the measuring reagent of the present invention. That is, the measuring reagent used therein includes an insoluble carrier particle group containing a plurality of insoluble carrier particles that support antibodies, the insoluble carrier particle group includes two or more types of insoluble carrier particle groups that are different in mean particle size from each other, in the two or more types of insoluble carrier particle groups that are different in mean particle size from each other, insoluble carrier particles contained in at least one type of insoluble carrier particle group support polyclonal antibodies and monoclonal antibodies, and insoluble carrier particles contained in another type of insoluble carrier particle group support at least one of the polyclonal antibodies and the monoclonal antibodies.

In the present invention, at least two types of insoluble carrier particle groups that are different in mean particle size from each other may be used. Preferably, two or more types of insoluble carrier particle groups are used that indicate different mean particle sizes from each other when, for example, the mean particle size is determined by a conventionally known predetermined method. In the present invention, the type of the "mean particle size" is not particularly limited, but, generally, examples thereof include a number mean diameter, length mean diameter, surface mean diameter, mass mean diameter, volume mean diameter, mean surface diameter, equivalent specific surface diameter, median diameter, and mode diameter. For measurements of those mean particle sizes, known measurement methods corresponding to them, respectively, can be employed. Furthermore, it also is possible to use two or more types of insoluble carrier particle groups that can be classified with two types of sieves whose opening sizes are different from each other.

Furthermore, in the present invention, a phrase "the insoluble carrier particle group includes two or more types of insoluble carrier particle groups that are different in mean particle size from each other" may denote that, for example, when the particle size distribution of all the insoluble carrier particles to be used is measured, at least two peaks are shown in a histogram. That is, in the present invention, a phrase "the insoluble carrier particle group including two or more types of insoluble carrier particle groups that are different in mean particle size from each other" also can be said that, for example, "the insoluble carrier particle group whose particle size distribution has at least two peaks". Furthermore, examples of the method of measuring the particle size distribution include a microscopy using an optical microscope or an electron microscope, a light scattering method, a light obscuration method, a laser diffractometry, an electrical resistance method, an electrical sensing zone method, a screening method, a liquid phase precipitation method, a centrifugal precipitation method, and an inertial impaction method. Furthermore, it also is possible to calculate the mean particle size from such a particle size distribution.

Furthermore, the two or more types of insoluble carrier particle groups that are contained in the insoluble carrier particle group and that are different in mean particle size from each other may contain, for example, particles whose particle size is mean particle size ± 15%, preferably particles whose particle size is mean particle size ± 5% on the basis of each mean particle size.

The types of the insoluble carrier particle groups that are different in mean particle size are not particularly limited as long as they are of two or more types. For example, they are of two to five types, preferably two to three types, and more preferably two types.

The mean particle size of the insoluble carrier particle group is not particularly limited and is, for example, 0.03 to 2 µm, preferably 0.05 to 1 µm, and more preferably 0.07 to 0.5 µm. In the present invention, for example, when two types of particle groups are used that are different in mean particle size from each other, the mean particle size of a group of particles whose mean particle size described above is small (hereinafter referred to as "small particles") is, for example, 0.03 to 0.2 µm, preferably 0.05 to 0.15 µm, and more preferably 0.07 to 0.12 µm, and in the same case, the mean particle size of a group of particles whose mean particle size described above is large (hereinafter referred to as "large particles") is not particularly limited and, for example, 0.08 to 2 µm, preferably 0.1 to 1 µm, and more preferably 0.12 to 0.5 µm.

The material for the insoluble carrier particles is not particularly limited and can be, for example, an organic compound such as a synthetic polymer or an inorganic compound such as porosity glass or silica gel. The form of the insoluble carrier particles is not particularly limited and is, for example, the form of latex particles, beads, or plates, preferably latex particles.

The material for the latex particles is not particularly limited and examples thereof include polystyrene, a styrene-butadiene copolymer, and a styrene-acrylate copolymer. Furthermore, in the latex particles, for example, a functional group may be added to each particle surface. Examples of the functional group include a carboxyl group, amino group, sulfo group, carbinol group, and amide group.

In the present invention, as described above, in the two or more types of insoluble carrier particle groups, insoluble carrier particles contained in at least one type of insoluble carrier particle group support both polyclonal antibodies and monoclonal antibodies, and insoluble carrier particles contained in another insoluble carrier particle group that is different in mean particle size support at least one of the polyclonal antibodies and the monoclonal antibodies. The latter particle group may support, for example, only one of the polyclonal antibodies and the monoclonal antibodies or both of them, preferably polyclonal antibodies or both of the polyclonal antibodies and the monoclonal antibodies, and more preferably both of them. In the present invention, in the case of using two types of insoluble carrier particle groups that are different in mean particle size from each other, for example, the small particles may be allowed to support both the polyclonal antibodies and the monoclonal antibodies and the large particles may be allowed to support at least one of the polyclonal antibodies and the monoclonal antibodies. On the contrary, for example, the small particles may be allowed to support at least one of the polyclonal antibodies and the monoclonal antibodies and the large particles may be allowed to support both the polyclonal antibodies and the monoclonal antibodies. Particularly, the small particles support preferably both the polyclonal antibodies and the monoclonal antibodies, and the large particles support preferably at least either one of the polyclonal antibodies or the monoclonal antibodies, and more preferably the polyclonal antibodies or both the polyclonal antibodies and the monoclonal antibodies.

In the insoluble carrier particles that support both the polyclonal antibodies and the monoclonal antibodies, the ratio between the polyclonal antibodies and the monoclonal antibodies to be supported is not particularly limited. Specifically, in the case of the small particles, the ratio of polyclonal antibodies : monoclonal antibodies is, for example, preferably 1:9 to 7:3 and more preferably 1:9 to 5:5. On the other hand, in the case of the large particles, the ratio of polyclonal antibodies : monoclonal antibodies is, for example, preferably 1:9 to 5:5. The ratio between the polyclonal antibodies and the monoclonal antibodies that the insoluble carrier particles support is, for example, a weight ratio or a ratio in the number of antibody molecules.

The support of the antibodies by the insoluble carrier particles is not particularly limited and, for example, can be achieved by sensitizing the particles with the antibodies. The sensitization reaction of the particles with the antibodies is not particularly limited and, for example, can be conducted suitably using a known method such as a physical adsorption method or a chemical bond method. Furthermore, when the surfaces of the insoluble carrier particles each have the aforementioned functional group added thereto, for example, the sensitization reaction may be performed after the functional group is activated.

In the sensitization reaction, the concentration of the insoluble carrier particles is not particularly limited. The concentration of the insoluble carrier particles denotes, for example, the concentration of the insoluble carrier particles contained in a sensitization reaction solution at the time of the reaction of sensitizing the insoluble carrier particles with the antibodies. The concentration of the insoluble carrier particles is, for example, 0.001 to 5 w/v%, preferably 0.01 to 2 w/v%, and more preferably 0.1 to 1 w/v%.

In the sensitization reaction, the concentration of the polyclonal antibodies or monoclonal antibodies to the insoluble carrier particles is not particularly limited and can be determined suitably according to, for example, the concentration of the insoluble carrier particles. The concentration of the antibodies denotes, for example, the concentration of the antibodies contained in the sensitization reaction solution at the time of reaction of sensitizing the insoluble carrier particles with the antibodies. When the large particles are allowed to support the polyclonal antibodies, for example, the concentration of the polyclonal antibodies in the reaction solution containing 1 w/v% insoluble carrier particles is, for example, 0∼2 mg/mL, preferably 0.001 to 2 mg/mL, more preferably 0.005 to 1.5 mg/mL, and further preferably 0.01 to 1 mg/mL. Furthermore, when the large particles are allowed to support the monoclonal antibodies, for example, the concentration of the monoclonal antibodies in the reaction solution containing 1 w/v% insoluble carrier particles is preferably 0 to 2 mg/mL, more preferably 0 to 1.5 mg/mL, and further preferably 0 to 1 mg/mL. On the other hand, when the small particles are allowed to support the polyclonal antibodies, for example, the concentration of the polyclonal antibodies in the reaction solution containing 1 w/v% insoluble carrier particles is, for example, 0 to 2 mg/mL, preferably 0.001 to 2 mg/mL, more preferably 0.005 to 1 mg/mL, and further preferably 0.01 to 0.5 mg/mL. Furthermore, when the small particles are allowed to support the monoclonal antibodies, for example, the concentration of the monoclonal antibodies in the reaction solution containing 1 w/v% insoluble carrier particles is, for example, 0 to 2 mg/mL, preferably 0.001 to 2 mg/mL, more preferably, 0.005 to 1.5 mg/mL, and further preferably 0.01 to 1 mg/mL.

When the insoluble carrier particles are allowed to support both the polyclonal antibodies and the monoclonal antibodies, the concentration of all the antibodies in the sensitization reaction is not particularly limited. When the small particles are allowed to support both the antibodies, for example, the concentration of all the antibodies in the reaction solution containing 1 w/v% insoluble carrier particles is, for example, 0.001 to 5 mg/mL, preferably 0.01 to 1 mg/mL, and more preferably 0.1 to 0.5 mg/mL. When the large particles are allowed to support both the antibodies, for example, the concentration of all the antibodies in the reaction solution containing 1 w/v% insoluble carrier particles is, for example, 0.001 to 5 mg/mL, preferably 0.01 to 2 mg/mL, and more preferably, 0.1 to 1 mg/mL.

As described above, when the insoluble carrier particles are allowed to support both the polyclonal antibodies and the monoclonal antibodies, the antibody concentration ratio between the polyclonal antibodies and the monoclonal antibodies with respect to the insoluble carrier particles in the sensitization reaction is not particularly limited. The antibody concentration ratio denotes, for example, the concentration ratio between both the antibodies (polyclonal antibodies : monoclonal antibodies) in the sensitization reaction solution in the reaction of sensitizing the insoluble carrier particles with both the antibodies. The antibody concentration ratio is, for example, a weight ratio. When the large particles are allowed to support the polyclonal antibodies and the monoclonal antibodies, for example, the antibody concentration ratio between the polyclonal antibodies and the monoclonal antibodies in the reaction solution containing 1 w/v% insoluble carrier particles is not particularly limited and is preferably, for example, 1:9 to 5:5. On the other hand, when the small particles are allowed to support the polyclonal antibodies and the monoclonal antibodies, for example, the concentration ratio between the polyclonal antibodies and the monoclonal antibodies in the reaction solution containing 1 w/v% insoluble carrier particles is, for example, preferably 1:9 to 7:3 and more preferably 1:9 to 5:5.

The antibodies are not particularly limited as long as they generate an antigen-antibody reaction with antigens to be measured. Examples thereof include antibody fragments such as Fab, Fab', and F(ab')₂, and immunoglobulin molecules. The polyclonal antibodies may be collected from blood serum derived from, for example, a mouse, rabbit, goat, sheep, or chicken by using a conventionally known method, or may be one of various commercially available antibodies. Furthermore, the monoclonal antibodies also are not particularly limited. For example, one of commercially available various antibodies may be used or one produced by a known method using, for example, a cell fusion technique or a gene recombination technique may be used suitably.

Next, with respect to the turbidimetric immunoassay of the present invention, an example of a specific method is described below but the turbidimetric immunoassay is not limited thereto.

First, using the aforementioned two or more types of insoluble carrier particle groups, the antibodies that the particles support and an object to be measured (antigens) in a specimen are allowed to undergo an antigen-antibody reaction and thereby the insoluble carrier particle groups are agglutinated.

The object to be measured (antigens) is not particularly limited as long as it generates an antigen-antibody reaction. Specific examples thereof include C-reactive protein, ASO (antistreptolysinO), a rheumatoid factor, HbA1c, creatinine, hepatitis virus, and various enzymes.

The specimen containing the object to be measured (antigens) is not particularly limited and examples thereof include common specimens in laboratory tests. Examples of the specimen include biological samples such as whole blood, hemocyte, blood serum, blood plasma, and urine. In the present invention, for example, the specimen that has been collected may be used as a measurement sample without any further processing or may be used as a measurement sample after being subjected to, for example, a dilution treatment, a filtration treatment, or a heat treatment beforehand prior to the antigen-antibody reaction. The solvent to be used for the dilution treatment is not particularly limited and examples thereof include water, saline, and a buffer solution. The buffer agent of the buffer solution is not particularly limited, for example, as long as it does not prevent, the aforementioned reaction. Examples thereof include MES (2-N-morpholinoethanesulfonic acid), phosphoric acid, and Tris. For example, other components may be added to the buffer solution. For the purpose of, for example, reactive stabilization, for instance, BSA may be added. Furthermore, the dilution rate of the specimen in the dilution treatment is not particularly limited and may be low in the case of, for example, measurement using such a test piece as described later. Specific examples of the dilution rate are, for example, preferably 5 to 100 times, more preferably 10 to 80 times, and further preferably 15 to 50 times.

The method of allowing the insoluble carrier particles that support the antibodies and the object to be measured (antigens) to undergo an antigen-antibody reaction is not particularly limited and can be, for example, a known method. For the reaction method, for example, a dispersion liquid in which the insoluble carrier particle groups are dispersed may be added to the measurement sample to allow a reaction to occur or the measurement sample may be added to the insoluble carrier particle groups to allow a reaction to occur. In the latter case, the insoluble carrier particle groups may be, for example, in the form of a dispersion liquid or in a dried state.
For example, a sample analysis tool such as a test piece, microchip, or cartridge as described later is applicable to the latter method described above. Specifically, the insoluble carrier particle groups are disposed in a reagent portion of a substrate, for example, in the form of a dispersion liquid or in a dried state and the measurement sample is added to the reagent portion. Thus an antigen-antibody reaction can be performed.

In the antigen-antibody reaction, the concentration of the insoluble carrier particles is not particularly limited. The concentration of the insoluble carrier particles denotes, for example, the concentration of the insoluble carrier particles in the antigen-antibody reaction solution at the time of the antigen-antibody reaction between the antibodies of the insoluble carrier particles and an object to be measured. The concentration of the insoluble carrier particles is, for example, preferably 0.011 to 1 w/v%, more preferably 0.033 to 0.5 w/v%, and further preferably 0.055 to 0.3 w/v%. Furthermore, in the concentration of the insoluble carrier particles, the aforementioned concentration of the particles of the small particle group is preferably 0.01 to 0.5 w/v%, more preferably 0.03 to 0.3 w/v%, and further preferably 0.05 to 0.2 w/v%. Moreover, in the concentration of the insoluble carrier particles, the concentration of the particles of the large particle group is preferably 0.001 to 0.05 w/v%, more preferably 0.003 to 0.04 w/v%, and further preferably 0.005 to 0.03 w/v%.

In the antigen-antibody reaction, the ratio between the particles of the small particle group and the particles of the large particle group is not particularly limited. Specifically, the weight ratio between the particles of the small particle group and the particles of the large particle group is preferably 100:1 to 1:100, more preferably 10:1 to 1:10, and further preferably 5:1 to 1:5.

A specific example of the conditions for the reaction solution for detecting CRP by immunoturbidimetry using blood as a specimen is indicated below but the present invention is not limited thereto. In the antigen-antibody reaction, the concentration of the insoluble carrier particles is not particularly limited. The concentration of the insoluble carrier particles denotes, for example, that of the insoluble carrier particles contained in an antigen-antibody reaction solution in the antigen-antibody reaction between the antibodies of the insoluble carrier particles and an object to be measured. The concentration of the insoluble carrier particles is, for example, preferably 0.011 to 1 w/v%, more preferably 0.033 to 0.5 w/v%, and further preferably 0.055 to 0.3 w/v%. Furthermore, in the concentration of the insoluble carrier particles, the concentration of the particles of the small particle group is preferably 0.01 to 0.5 w/v%, more preferably 0.03 to 0.3 w/v%, and further preferably 0.05 to 0.2 w/v%. Moreover, in the concentration of the insoluble carrier particles, the concentration of the particles of the large particle group is preferably 0.001 to 0.05 w/v%, more preferably 0.003 to 0.04 w/v%, and further preferably 0.005 to 0.03 w/v%.

Subsequently, the aforementioned antigen-antibody reaction is measured. That is, the antigen-antibody reaction is measured, for example, through detection of the agglutination degree of the insoluble carrier particle group, which is generated by the antigen-antibody reaction. This allows the quantity of the object to be measured to be determined indirectly.

The method of measuring the antigen-antibody reaction is not particularly limited and can be, for example, a method in which, for example, scattered light or the absorbance of a reaction solution after the antigen-antibody reaction is measured by an optical method. For example, a general optical measuring instrument can be used for the optical method. Furthermore, the method of evaluating measurement results also is not particularly limited. For example, the variation in absorbance within a predetermined period of time after initiation of the antigen-antibody reaction may be employed as an index. In this case, the predetermined period of time after initiation of the reaction is not particularly limited and is preferably 0 to 5 minutes, more preferably 30 to 180 seconds, and further preferably 30 to 150 seconds after the initiation of the reaction.

The quantitative determination can be carried out based on, for example, a calibration curve of a standard sample containing an object to be measured with a known concentration. Specifically, first, a plurality of standard samples set in a target concentration range are prepared and are allowed to undergo an antigen-antibody reaction under the same conditions, and thereby the degree to which they have been agglutinated is detected. Thereafter, a calibration curve is created from the known concentration and measured values of, for example, absorbance that indicate the agglutination degree, and the measured value with respect to the specimen is assigned to the calibration curve, so that the object to be measured in the specimen can be determined in quantity.

### <Measuring Reagent>

The measuring reagent of the present invention is a measuring reagent that is used for the turbidimetric immunoassay of the present invention and that contains the aforementioned insoluble carrier particle group. The measuring reagent of the present invention is not limited as long as it contains the aforementioned insoluble carrier particle group, and other compositions and conditions are not limited by any means. In this context, the measuring reagent is as described with respect to the turbidimetric immunoassay of the present invention unless otherwise indicated particularly.

In the present invention, the respective ratios of the two or more types of insoluble carrier particle groups contained are not particularly limited. However, preferably, the respective particle groups are contained so that, for example, when the measuring reagent of the present invention is used for the turbidimetric immunoassay, the ratios of the respective particles groups at the time of the antigen-antibody reaction satisfy the conditions described with respect to the turbidimetric immunoassay of the present invention.

The measuring reagent of the present invention may contain, for example, a buffer agent as described above, dispersant, or stabilizing agent in addition to the insoluble carrier particle groups. Furthermore, the measuring reagent of the present invention may be, for example, a dried material or a liquid.

### <Sample Analysis Tool>

The sample analysis tool of the present invention is characterized by including a measuring reagent of the present invention as described above. Specifically, the sample analysis tool of the present invention includes a substrate and a measuring reagent and is used for a turbidimetric immunoassay, with the measuring reagent being disposed on the substrate,
wherein the measuring reagent is a measuring reagent of the present invention. As long as the sample analysis tool of the present invention contains a measuring reagent of the present invention, other configurations and conditions are not limited by any means. In the sample analysis tool of the present invention, the measuring reagent may be disposed as, for example, a dried material or a liquid (dispersion liquid) according to the form thereof.

The form of the sample analysis tool according to the present invention is not limited by any means and examples thereof include a cartridge, a test piece, and a chip such as a microchip. Preferably, the sample analysis tool can be connected to, for example, a general measuring instrument for detecting the degree of agglutination obtained through the aforementioned antigen-antibody reaction. The form of the cartridge is, for example, one in which the substrate is provided with a reagent reservoir with the measuring reagent disposed therein. In the cartridge, for example, before or after it is connected to the measuring instrument, a measurement sample is introduced into the reagent reservoir with the measuring reagent disposed therein, and the degree of agglutination inside the reagent reservoir can be measured with the measuring instrument after the antigen-antibody reaction. Furthermore, in the aforementioned cartridge, for example, the substrate further may have a reaction reservoir that is connected to the reagent reservoir through a channel. In this case, a measurement sample may be introduced into the reagent reservoir, a mixed solution of the measurement sample and the measuring reagent may be transferred to the reaction reservoir through the channel, and thereby the agglutination degree may be measured inside the reaction reservoir. The measuring reagent of the present invention disposed in the reagent reservoir of the cartridge may be, for example, a liquid (dispersion liquid) or a dried material. Moreover, the form of the test piece is, for example, one in which a reagent portion with a measuring reagent of the present invention is disposed on, for example, a strip-shaped substrate. Examples of the material for the reagent portion include filter paper and porous bodies made of various polymers. In such a test piece, for example, a measurement sample is added to the reagent portion with the measuring reagent disposed therein, and the agglutination degree in the reagent portion can be measured with the measuring instrument after the antigen-antibody reaction. Preferably, the measuring reagent of the present invention in each test piece is present, for example, in a dried state. For example, before or after, for example, the porous body is disposed on the substrate, the measuring reagent in the form of a dispersion liquid may be fed to, for example, the porous body and this may then be dried. The form of the chip such as a microchip is, for example, one in which the substrate has a sample introducing portion, a reagent portion, and a channel, and the introducing portion and the reagent portion are connected to each other through the channel. In such a chip, for example, a measurement sample is introduced from the introducing portion, the measurement sample is transferred to the reagent portion through the channel, the antigen-antibody reaction is performed in the reagent portion, and thereafter, the agglutination degree in the reagent portion can be measured with the measuring instrument. The measuring reagent of the present invention disposed in the reagent portion of the chip may be, for example, a liquid or a dried material. Furthermore, in such a sample analysis tool, for example, the reagent portion or reagent reservoir further may contain a detection reagent for detecting an agglutinate as required.

Hereinafter, examples of the present invention are described together with comparative examples. The present invention is neither limited nor restricted by the following examples or comparative examples.

### Examples

### [Example 1]

In this example, a measuring reagent was prepared by the following steps A and B using, as insoluble carrier particle groups that were different in mean particle size from each other, a small particle group that was allowed to support monoclonal antibodies and polyclonal antibodies and a large particle group that was allowed to support polyclonal antibodies.

### A. Preparation of insoluble carrier particles

In this example, carboxyl group denatured polystyrene latex particles (manufactured by Seradyn, Inc.) with a mean particle size of 0.096 µm were used as insoluble carrier particles (small particles) of the small particle group, and carboxyl group denatured polystyrene latex particles (manufactured by Seradyn, Inc.) with a mean particle size 0.213 µm were used as insoluble carrier particles (large particles) of the large particle group. First, an activation solution with the following composition containing the small particles was mixed while being stirred, and thereby was allowed to react at room temperature (25°C) for one hour. Thus, the functional groups of the particle surfaces were activated. After completion of the reaction, this was centrifuged (6000 rpm, 15 minutes) and a supernatant was removed. The small particles with the functional groups that had been activated were suspended in 4 mL of 50 mmol/L MES buffer (pH 6.1) and then were redispersed ultrasonically to be washed. After the washing operation was performed again, it was centrifuged (6000 rpm, 15 minutes) and the supernatant was removed. The resultant small particles were suspended using 50 mmol/L MES buffer (pH 6.1) and were redispersed ultrasonically, and thereby a latex dispersion liquid of the small particles was obtained.
The concentration of the small particles in the latex dispersion liquid was 2 w/v%. On the other hand, a latex dispersion liquid of large particles also was prepared in the same manner as in the case of preparing the latex dispersion liquid of small particles except that the large particles were used as the carboxyl group denatured polystyrene latex particles.

| <Composition of activation solution> | |
|---|---|
| Reagent | Final Concentration |
| MES (pH 6.1, DOJINDO LABORATORIES) | 50 mmol/L |
| NHS aqueous solution *1 | 100 mmol/L |
| EDAC aqueous solution *2 | 1.0 mmol/L |
| Carboxyl group denatured polystyrene latex particles | 1 w/v% |
| (10% solution) | |

| | |
|---|---|
| *1 NHS (N-hydroxysuccinimide, manufactured by Nacalai Tesque, Inc.) *2 EDAC (1-ethyl-3-(3-dimethylaminopropyl)-carbodiimide hydrochloride, manufactured by SIGMA) | |

Next, 2 mL of antibody solution for sensitizing small particles with the following composition was added to 2 mL of latex dispersion liquid of the small particles while this was stirred in such a manner as not to induce bubbling. This was allowed to react at room temperature (25°C) for one hour and thereby the small particles were subjected to a sensitization reaction. The concentrations indicated for the following composition are final concentrations of the respective components in the sensitization reaction solution. The final concentration of the small particles in the sensitization reaction solution was 1 w/v%. Furthermore, in the sensitization reaction solution, the concentration ratio (weight ratio) between the polyclonal antibodies and the monoclonal antibodies that the small particles are allowed to support, i.e. polyclonal antibodies : monoclonal antibodies, was 7:3.

| <Composition of antibody solution for sensitizing small particles> | |
|---|---|
| Composition | Final Concentration in sensitization reaction |
| MES (pH 6.1, DOJINDO LABORATORIES) | 50 mmol/L |
| BSA (manufactured by SIGMA) | 1 mg/mL |
| Monoclonal antibody solution *1 | 0.12 mg/mL |
| Polyclonal antibody solution *2 | 0.28 mg/mL |

| | |
|---|---|
| *1 Rabbit anti-human CRP monoclonal antibody solution (Antibody concentration: 10.5 mg/mL, Clone No. 8, manufactured by Immuno Probe Co., Ltd.) *2 Rabbit anti-human CRP polyclonal antibody solution (Protein concentration: 12.2 mg/mL, Becker titer 3.5 mg/mL, manufactured by Oriental Yeast Co., Ltd.) | |

After completion of the reaction, it was centrifuged (6000 rpm, 15 minutes) and a supernatant was removed. The small particles that had been sensitized were suspended in 4 mL of 50 mmol/L MES buffer (pH 6.1) and were dispersed ultrasonically to be washed. This was centrifuged (6000 rpm, 15 minutes) again and a supernatant was removed. Thereafter, the small particles that had been washed were suspended in 2 mL of 50 mmol/L MES buffer (pH 6.1) and were redispersed ultrasonically. The small particles thus redispersed were mixed with 2 mL of blocking buffer (50 mmol/L MES buffer (pH 6.1), 4 w/v% BSA) and were then dispersed ultrasonically. Thereafter, this was allowed to stand for one night at 4°C and a blocking treatment was then performed. After completion of the blocking treatment, it was centrifuged (6000 rpm, 15 minutes) and a supernatant was removed. Using a dispersion liquid having the following composition, the small particles were then dispersed so that the dispersion concentration of the small particles was 3.33 w/v%.

| <Composition of dispersion liquid> | |
|---|---|
| Composition | Concentration |
| Tris-HCL (pH 7.5, manufactured by Nacalai Tesque, Inc.) | 50 mmol/L |
| BSA (manufactured by SIGMA) | 1 w/v% |
| Saponin (manufactured by Nacalai Tesque, Inc.) | 2.14 w/v% |
| D(-)mannitol (manufactured by Nacalai Tesque, Inc.) | 2 w/v% |

On the other hand, the large particles were subjected to a sensitization reaction in the same manner as in the case of the small particles except that an antibody solution for sensitizing large particles with the following composition was used, the final concentration of the large particles in the sensitization reaction solution was 0.5 w/v%, the final concentration of the polyclonal antibodies was 0.5 mg/mL, and no monoclonal antibodies were added. Furthermore, the large particles were dispersed in a dispersion liquid in the same manner as in the case of the small particles except that the dispersion concentration of the large particles was 1.67 w/v%. In the sensitization reaction solution, the concentration ratio (weight ratio) between the polyclonal antibodies and the monoclonal antibodies that the large particles were allowed to support, i.e. polyclonal antibodies : monoclonal antibodies, was 10:0. The concentrations indicated for the following composition denote the final concentrations of the respective components of the sensitization reaction solution.

| <Composition of antibody solution for sensitizing large particles> | |
|---|---|
| Composition | Final Concentration in sensitization reaction |
| MES (pH 6.1, DOJINDO LABORATORIES) | 50 mmol/L |
| BSA (manufactured by SIGMA) | 1 mg/mL |
| Polyclonal antibody solution * | 0.5 mg/mL |

| | |
|---|---|
| * Rabbit anti-human CRP polyclonal antibody solution (Protein concentration: 12.2 mg/mL, Becker titer 3.5 mg/mL, manufactured by Oriental Yeast Co., Ltd.) | |

### B. Preparation of measuring reagent

First, a dispersion liquid containing the small particles and a dispersion liquid containing the large particles that had been prepared were mixed together so that the concentration ratio (weight ratio) between the small particles and the large particles was 10:1. Thus, a latex mixed solution was obtained. The latex mixed solution then was diluted with the above-mentioned dispersion liquid so that the concentration of the small particles was 0.372 w/v% and the concentration of the large particles was 0.036 w/v%. Thus, a measuring reagent of this example was prepared. In the latex mixed solution, the weight ratio between the small particles and the large particles, i.e. small particles : large particles, was 10:1.

### [Example 2]

The measuring reagent of this example was prepared in the same manner as in Example 1 except that in the sensitization reaction of the small particles, the final concentrations of the polyclonal antibody solution and monoclonal antibody solution in the sensitization reaction solution each were 0.2 mg/mL. In the sensitization reaction solution, the concentration ratio (weight ratio) of the antibodies that the small particles were allowed to support, i.e. polyclonal antibodies : monoclonal antibodies, was 5:5.

### [Example 3]

The measuring reagent of this example was prepared in the same manner as in Example 1 except that in the sensitization reaction of the small particles, the final concentrations of the polyclonal antibody solution and monoclonal antibody solution in the sensitization reaction solution were 0.12 mg/mL and 0.28 mg/mL, respectively. In the sensitization reaction solution, the concentration ratio (weight ratio) of the antibodies that the small particles were allowed to support, i.e. polyclonal antibodies : monoclonal antibodies, was 3:7.

### [Example 4]

The measuring reagent of this example was prepared in the same manner as in Example 1 except that in the sensitization reaction of the small particles, the final concentrations of the polyclonal antibody solution and monoclonal antibody solution in the sensitization reaction solution were 0.04 mg/mL and 0.36 mg/mL, respectively. In the sensitization reaction solution, the concentration ratio (weight ratio) of the antibodies that the small particles were allowed to support, i.e. polyclonal antibodies : monoclonal antibodies, was 1 : 9.

### [Comparative Example 1]

In the measuring reagent of this example, the small particles were allowed to support only polyclonal antibodies. Specifically, the measuring reagent of this example was prepared in the same manner as in Example 1 except that in the sensitization reaction of the small particles, the final concentration of the polyclonal antibodies in the sensitization reaction solution was 0.4 mg/mL (no monoclonal antibodies were added). In the sensitization reaction solution, the concentration ratio (weight ratio) of the antibodies that the small particles were allowed to support, i.e. polyclonal antibodies : monoclonal antibodies, was 10:0.

### [Comparative Example 2]

In the measuring reagent of this example, the small particles were allowed to support only monoclonal antibodies. Specifically, the measuring reagent of this example was prepared in the same manner as in Example 1 except that in the sensitization reaction of the small particles, the final concentration of the monoclonal antibody in the sensitization reaction solution was 0.4 mg/mL (no polyclonal antibodies were added). In the sensitization reaction solution, the concentration ratio of the antibodies that the small particles were allowed to support, i.e. polyclonal antibodies : monoclonal antibodies, was 0:10.

Using the measuring reagents of Examples 1 to 4 and Comparative Examples 1 and 2, CRP was measured by turbidimetric immunoassay as follows. For the CRP, C-Reactive Protein Antigen, High Pure (Manufactured by Capricon) was used.

That is, first, the above-mentioned CRP was diluted with a diluent (50 mmol/L Tris-HCl (pH 7.5), 100 mmol/L NaCl (manufactured by Nacalai Tesque, Inc.), 1 w/v% BSA) and thereby CRP solutions (specimens) with predetermined concentrations (0, 0.05, 0.1, 0.15, 0.5, 1, 2.5, 5, 10, and 20 mg/100 mL) were prepared. Thereafter, the above-mentioned respective CRP solutions (specimens) were diluted 1.66 times with the aforementioned diluent, and thus measurement samples were prepared. Then 63 µL of reaction buffer (1.28 w/v% NaCl) and 21 µL of each of the above-mentioned measuring reagents were added to 6 µL of each of the above-mentioned measurement samples and they were allowed to react at 37°C. For a period of five minutes from the initiation of the reaction (addition), absorbance at 658 nm of the reaction solution was measured using an automatic general measuring instrument (trade name: Bio-majesty (BM-8), manufactured by JEOL Ltd.). Thereafter, the absorbance measured value obtained 30 seconds after the initiation of the reaction was subtracted from that obtained 150 seconds after the initiation of the reaction and thereby the variation in absorbance was calculated. This variation was used for a measurement evaluation. With respect to the concentration of latex particles in the reaction solution, the concentration of the small particles was 0.087 w/v%, that of the large particles was 0.008 w/v%, and that of all the latex particles was 0.095 w/v%.

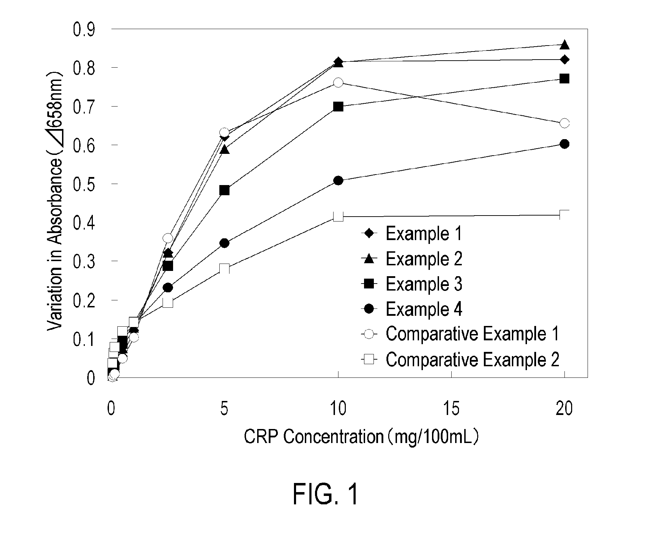

The measurement results obtained using the measuring reagents of Examples 1 to 4 and Comparative Examples 1 and 2 are shown in the graph in FIG. 1. In the graph, the horizontal axis indicates the concentration (mg/100 mL) of CRP in each CRP solution (specimen), and the vertical axis indicates the variation in absorbance at each CRP concentration. In the graph shown in FIG. 1, the respective examples and comparative examples are plotted as follows: Example 1 with ◆, Example 2 with A, Example 3 with ■, Example 4 with ●, Comparative Example 1 with ○, and Comparative Example 2 with □. As shown in FIG. 1, when the measuring reagents of Examples 1 to 4 were used in which the small particles were allowed to support both the polyclonal antibodies and the monoclonal antibodies, the variation in absorbance increased with an increase in CRP concentration.
On the other hand, in the case where the measuring reagents of the comparative examples were used in which the small particles were allowed to support only one of the antibodies, when the CRP concentration exceeded 10 mg/100 mL, the variation in absorbance hardly was changed in Comparative Example 2. On the contrary, it was decreased in Comparative Example 1. Thus, it was proved that when the small particles were allowed to support both the polyclonal antibodies and the monoclonal antibodies, measurement with high sensitivity was possible in a wide concentration range from a low concentration to a high concentration. As shown in the measurement results obtained using measuring reagents of Examples 1 to 4, it was possible to adjust the sensitivity easily by changing the ratio between the polyclonal antibodies and the monoclonal antibodies that were supported.
Furthermore, although the dilution rate of the CRP solution (specimen) in the reaction solution was very low, specifically, about 25 times, the variation in absorbance increased with an increase in CRP concentration when the measuring reagents of Examples 1 to 4 were used, and good measurement results were obtained. Furthermore, in the measurement performed using the measuring reagents of Examples 1 to 4 with decreased ratios of the monoclonal antibodies to be supported, the amount of increase in absorbance was larger as compared to the case of using the measuring reagent of Comparative Example 2 in which only the monoclonal antibodies were supported, and the measurement sensitivity was improved in each case.

### [Example 5]

In this example, the sensitization reaction of the small particles was performed in the same manner as in Example 1 except that the final concentrations of the polyclonal antibody solution and monoclonal antibody solution in the sensitization reaction solution were 0.04 mg/mL and 0.36 mg/mL, respectively. In the sensitization reaction solution, the concentration ratio (weight ratio) of the antibodies that the small particles were allowed to support, i.e. polyclonal antibodies : monoclonal antibodies, was 1:9. On the other hand, the sensitization reaction of the large particles was performed in the same manner as in Example 1 except that the final concentrations of the polyclonal antibody solution and monoclonal antibody solution in the sensitization reaction solution each were 0.25 mg/mL. In the sensitization reaction solution, the concentration ratio (weight ratio) of the antibodies that the large particles were allowed to support, i.e. polyclonal antibodies : monoclonal antibodies, was 5:5. The measuring reagent of this example was prepared in the same manner as in Example 1 except that a dispersion liquid containing small particles and a dispersion liquid containing large particles were mixed so that the concentration ratio (weight ratio) between the small particles and the large particles was 5:1, and further it was diluted with the dispersion liquid so that the concentrations of the small particles and the large particles were 0.417 w/v% and 0.083 w/v%, respectively.

### [Example 6]

In this example, the sensitization reaction of the small particles was performed in the same manner as in Example 1 except that the final concentrations of the polyclonal antibody solution and monoclonal antibody solution in the sensitization reaction solution were 0.04 mg/mL and 0.36 mg/mL, respectively. In the sensitization reaction solution, the concentration (weight ratio) of the antibodies that the small particles were allowed to support, i.e. polyclonal antibodies : monoclonal antibodies, was 1:9. On the other hand, the sensitization reaction of the large particles was performed in the same manner as in Example 1 except that the final concentrations of the polyclonal antibody solution and monoclonal antibody solution in the sensitization reaction solution were 0.15 mg/mL and 0.35 mg/mL, respectively. In the sensitization reaction solution, the concentration ratio (weight ratio) of the antibodies that the large particles were allowed to support, i.e. polyclonal antibodies : monoclonal antibodies, was 3:7. The measuring reagent of this example was prepared in the same manner as in Example 1 except that a dispersion liquid containing small particles and a dispersion liquid containing large particles were mixed so that the concentration ratio (weight ratio) between the small particles and the large particles was 5:1, and further it was diluted with the dispersion liquid so that the concentrations of the small particles and the large particles were 0.417 w/v% and 0.083 w/v%, respectively.

### [Example 7]

In this example, the sensitization reaction of the small particles was performed in the same manner as in Example 1 except that the final concentrations of the polyclonal antibody solution and monoclonal antibody solution in the sensitization reaction solution were 0.04 mg/mL and 0.36 mg/mL, respectively. In the sensitization reaction solution, the concentration ratio (weight ratio) of the antibodies that the small particles were allowed to support, i.e. polyclonal antibodies : monoclonal antibodies, was 1:9. On the other hand, the sensitization reaction of the large particles was performed in the same manner as in Example 1 except that the final concentrations of the polyclonal antibody solution and monoclonal antibody solution in the sensitization reaction solution were 0.1 mg/mL and 0.4 mg/mL, respectively. In the sensitization reaction solution, the concentration ratio (weight ratio) of the antibodies that the large particles were allowed to support, i.e. polyclonal antibodies : monoclonal antibodies, was 2:8. The measuring reagent of this example was prepared in the same manner as in Example 1 except that a dispersion liquid containing small particles and a dispersion liquid containing large particles were mixed so that the concentration ratio (weight ratio) between the small particles and the large particles was 5:1, and further it was diluted with the dispersion liquid so that the concentrations of the small particles and the large particles were 0.417 w/v% and 0.083 w/v%, respectively.

### [Example 8]

In this example, the sensitization reaction of the small particles was performed in the same manner as in Example 1 except that the final concentrations of the polyclonal antibodies and monoclonal antibody solution in the sensitization reaction solution were 0.04 mg/mL and 0.36 mg/mL, respectively. In the sensitization reaction solution, the concentration ratio (weight ratio) of the antibodies that the small particles were allowed to support, i.e. polyclonal antibodies : monoclonal antibodies, was 1:9. On the other hand, the sensitization reaction of the large particles was performed in the same manner as in Example 1 except that the final concentrations of the polyclonal antibody solution and monoclonal antibody solution in the sensitization reaction solution were 0.05 mg/mL and 0.45 mg/mL, respectively. In the sensitization reaction solution, the concentration ratio (weight ratio) of the antibodies that the large particles were allowed to support, i.e. polyclonal antibodies : monoclonal antibodies, was 1:9. The measuring reagent of this example was prepared in the same manner as in Example 1 except that a dispersion liquid containing small particles and a dispersion liquid containing large particles were mixed so that the concentration ratio (weight ratio) between the small particles and the large particles was 5:1, and further it was diluted with the dispersion liquid so that the concentrations of the small particles and the large particles were 0.417 w/v% and 0.083 w/v%, respectively.

### [Example 9]

In this example, the sensitization reaction of the small particles was performed in the same manner as in Example 1 except that the final concentrations of the polyclonal antibodies and monoclonal antibody solution in the sensitization reaction solution were 0.04 mg/mL and 0.36 mg/mL, respectively. In the sensitization reaction solution, the concentration ratio (weight ratio) of the antibodies that the small particles were allowed to support, i.e. polyclonal antibodies : monoclonal antibodies, was 1:9. On the other hand, the sensitization reaction of the large particles was performed in the same manner as in Example 1 except that the final concentration of the polyclonal antibody solution in the sensitization reaction solution was 0.5 mg/mL and no monoclonal antibodies were added. In the sensitization reaction solution, the concentration ratio (weight ratio) of the antibodies that the large particles were allowed to support, i.e. polyclonal antibodies : monoclonal antibodies, was 10:0. The measuring reagent of this example was prepared in the same manner as in Example 1 except that a dispersion liquid containing small particles and a dispersion liquid containing large particles were mixed so that the concentration ratio (weight ratio) between the small particles and the large particles was 5:1, and further it was diluted with the dispersion liquid so that the concentrations of the small particles and the large particles were 0.417 w/v% and 0.083 w/v%, respectively.

The measurements were carried out by turbidimetric immunoassay in the same manner as in Examples 1 to 4 and Comparative Examples 1 and 2 described above except that each of the measuring reagents of Examples 5 to 9 was used as the aforementioned measuring agent, and with respect to the concentration of the latex particles in the reaction solution at the time of an antigen-antibody reaction, the concentrations of the small particles and the large particles were 0.097 w/v% and 0.019 w/v%, respectively (the concentration of all the latex particles was 0.116 w/v%).

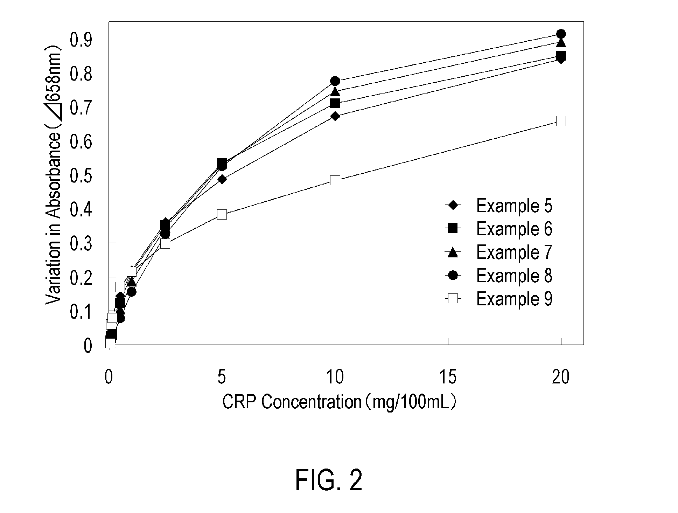

The measurement results obtained using the measuring reagents of Examples 5 to 9 are shown in the graph in FIG. 2. In the graph, the horizontal axis indicates the concentration (mg/100 mL) of CRP in each CRP solution (specimen), and the vertical axis indicates the variation in absorbance at each CRP concentration. In the graph, the respective examples are plotted as follows: Example 5 with ◆, Example 6 with ■, Example 7 with ▲, Example 8 with ●, and Example 9 with □. As shown in FIG. 2, in any cases of using the measuring reagents of Examples 5 to 9, the variation in absorbance increased with an increase in CRP concentration, and the measurement was possible in a wide concentration range. Furthermore, in the measurements performed using the measuring reagents of Examples 5 to 8 in which large particles that were allowed to support both the antibodies were used, the measurement sensitivity in a high concentration range further was improved as compared to that obtained in the measurement using the measuring reagent of Example 9 in which only the polyclonal antibodies were supported.

### [Example 10]

In this example, the sensitization reaction of the small particles was performed in the same manner as in Example 1 except that the final concentrations of the polyclonal antibodies and monoclonal antibodies in the sensitization reaction solution were 0.04 mg/mL and 0.36 mg/mL, respectively. In the sensitization reaction solution, the concentration ratio (weight ratio) of the antibodies that the small particles were allowed to support, i.e. polyclonal antibodies : monoclonal antibodies, was 1:9. On the other hand, the sensitization reaction of the large particles was performed in the same manner as in Example 1. In the sensitization reaction solution, the concentration ratio (weight ratio) of the antibodies that the large particles were allowed to support, i.e. polyclonal antibodies : monoclonal antibodies, was 10:0. Furthermore, in this example, a dispersion liquid containing small particles and a dispersion liquid containing large particles were prepared in the same manner as in Example 1 except that a dispersion liquid having the following composition was used and the small particles and the large particles were dispersed in the dispersion liquid so that the concentrations of the small particles and large particles were 2 w/v% and 0.4 w/v%, respectively. Thereafter, the dispersion liquid of the small particles and the dispersion liquid of the large particles were mixed at a volume ratio of 1:1. Thus a measuring reagent having the following composition was prepared. In the measuring reagent of this example, the concentration ratio (weight ratio) between the small particles and the large particles was 5:1.

| <Composition of dispersion liquid> | |
|---|---|
| Composition | Concentration |
| Tris-HCL (pH 7.5, manufactured by Nacalai Tesque, Inc.) | 200 mmol/L |
| BSA (manufactured by SIGMA) | 4 w/v% |
| Saponin (manufactured by Nacalai Tesque, Inc.) | 8.56 w/v% |
| Sucrose (manufactured by Nacalai Tesque, Inc.) | 4 w/v% |

| <Composition of measuring reagent> | |
|---|---|
| Composition | Concentration |
| Tris-HCL (pH 7.5, manufactured by Nacalai Tesque, Inc.) | 100 mmol/L |
| BSA (manufactured by SIGMA) | 2 w/v% |
| Saponin (manufactured by Nacalai Tesque, Inc.) | 4.28 w/v% |
| Sucrose (manufactured by Nacalai Tesque, Inc.) | 2 w/v% |
| Small particles (0.096 µm) | 1 w/v% |
| Large particles (0.213 µm) | 0.2 w/v% |

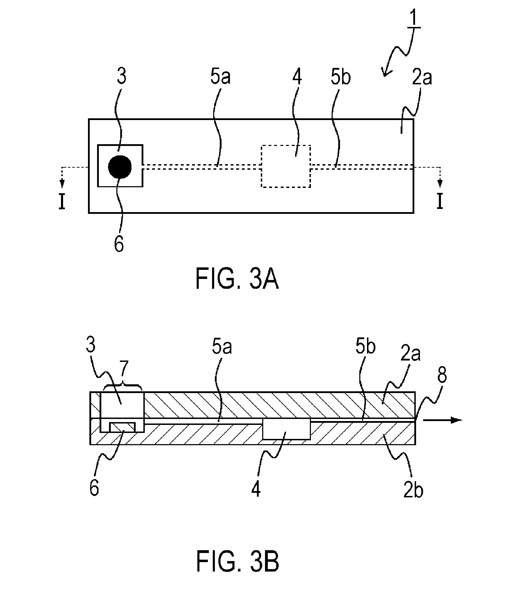

Next, a sample analysis tool 1 of this example described below was produced. FIG. 3 shows the sample analysis tool 1 of this example. FIG. 3A is a plan view of the sample analysis tool 1, and FIG. 3B is a cross-sectional view that is viewed from the I-I direction of the sample analysis tool 1 shown in FIG. 3A. FIGS. 3A and 3B are schematic views of the sample analysis tool 1 according to this example, and, for example, the sizes and ratios of the whole and the respective components are different from actual ones. The sample analysis tool 1 includes an upper substrate 2a and a lower substrate 2b and the upper substrate 2a is stacked on the lower substrate 2b. The lower substrate 2b has concave portions near the left end and the center in the longitudinal direction thereof, respectively. The former serves as a reagent portion 3 where a measuring reagent 6 is disposed. The latter serves as a measurement portion 4 where a reaction solution is measured. In the lower substrate 2b, grooves are formed between the reagent portion 3 and the measurement portion 4 and between the measurement portion 4 and the right end in the longitudinal direction, respectively. The grooves form channels 5a and 5b when the upper substrate 2a is stacked thereon. Furthermore, in the sample analysis tool 1, an opening 8 located at the right end of the channel 5b is a connection part for a negative pressure generator (for instance, a pipette) for allowing the insides of the channels 5a and 5b to have negative pressure. On the other hand, the upper substrate 2a has a through-hole 7 in a position that is in the vicinity of the left end in the longitudinal direction and that corresponds to the reagent portion 3 of the lower substrate 2b. This serves as an introducing portion 7 for a measurement sample. The material for the upper substrate 2a is PET (polyethylene terephthalate) and the material for the lower substrate 2b is PMMA (polymethyl methacrylate).

In this example, first, the upper substrate 2a was stacked on the lower substrate 2b and 2.45 µL of the measuring reagent was added to the reagent portion 3 of the lower substrate 2b through the introducing portion 7 using a pipette. This was dried at room temperature for two hours and was then allowed to stand in an environment with a humidity of 1% or lower for three days. Thus, the sample analysis tool 1 was produced. The sample analysis tool 1 was kept in an aluminum pack containing silica gel until the time of use.

Blood was collected into a blood collection tube and then was centrifuged. Thereafter, blood plasma was removed. The resultant hemocyte was washed three times with saline, and thereby washed hemocyte was prepared. On the other hand, CRP was added to CRP free serum (manufactured by Biopool) so that predetermined concentrations (0, 0.2, 0.4, 20, 40, and 80 mg/100 mL) were obtained. Thus, CRP solutions were prepared. Thereafter, 50 µL of the CRP solution was added to 60 µL of the CRP free serum and 90 µL of the washed hemocyte and thereby the total was 200 µL. Then 25 µL of 200 µL of the mixed solution further was diluted 25 times using 600 µL of saline and thereby a CRP-washed hemocyte solution was prepared. These were used as measurement samples. The CRP-washed hemocyte solutions had CRP concentrations of 0, 0.05, 0.1, 5, 10, and 20 mg/100 mL, respectively. 21 µL of each CRP-washed hemocyte solution was dropped into the reagent portion 3, and the measuring reagent 6 that had been dried was suspended. Immediately thereafter, with a pipette connected to the opening 8 at the end of the channel 5b, suction was performed in the direction indicated with an arrow in FIG. 3(B) to allow the inside of the channel 5b to have negative pressure, and thereby the suspension was transferred into the measurement portion 4 to allow the CRP to be reacted with the measuring reagent of this example. With respect to the concentration of the insoluble carrier particles in the suspension at the time of the reaction, the concentration of small particles was 0.117 w/v%, that of the large particles was 0.023 w/v%, and that of all the particles was 0.14 w/v%. The time at which the suspension was transferred to the measurement portion 4 is taken as reaction initiation. The absorbance of the measurement portion 4 at a measurement wavelength of 660 nm was measured one minute and four minutes after the reaction initiation using a spectrophotometer. The absorbance measured one minute after the reaction initiation was subtracted from the absorbance measured four minutes after the reaction initiation. Thus the variation in absorbance over three minutes was calculated.

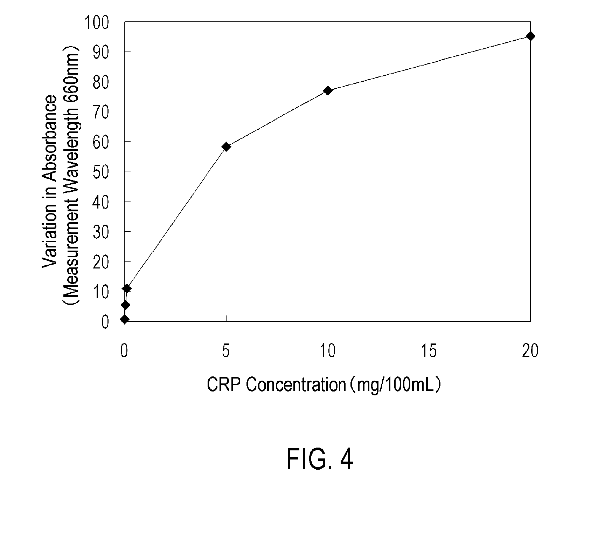

The graph shown in FIG. 4 indicates measurement results of the variations in absorbance. In FIG. 4, the vertical axis indicates the variation in absorbance and the horizontal axis indicates the CRP concentration (mg/100 mL) in the CRP-washed hemocyte solution. As shown in FIG. 4, in the measurement using the sample analysis tool 1 of this example, the variation in absorbance increased with an increase in CRP concentration and thus the measurement was possible in a wide concentration range.

### Industrial Applicability

The present invention is applicable to, for example, laboratory tests and various screenings. The applications thereof are not limited and the present invention is applicable to a wide range of fields.

## Claims

1. A measuring reagent that is used for a turbidimetric immunoassay,
wherein the measuring reagent comprises an insoluble carrier particle group containing a plurality of insoluble carrier particles that support antibodies,
the insoluble carrier particle group comprises two or more types of insoluble carrier particle groups that are different in mean particle size from each other, and
in the two or more types of insoluble carrier particle groups that are different in mean particle size from each other,
insoluble carrier particles contained in at least one type of insoluble carrier particle group support polyclonal antibodies and monoclonal antibodies, and
insoluble carrier particles contained in another type of insoluble carrier particle group support at least one of the polyclonal antibodies and the monoclonal antibodies.

2. The measuring reagent according to claim 1, wherein the insoluble carrier particle group comprises two types of insoluble carrier particle groups that are different in mean particle size from each other, and
in the two types of insoluble carrier particle groups,
insoluble carrier particles of an insoluble carrier particle group with a small mean particle size support polyclonal antibodies and monoclonal antibodies, and
insoluble carrier particles of an insoluble carrier particle group with a large mean particle size support at least one of the polyclonal antibodies and the monoclonal antibodies.

3. The measuring reagent according to claim 1, wherein the insoluble carrier particle group comprises two types of insoluble carrier particle groups that are different in mean particle size from each other, and
in the two types of insoluble carrier particle groups,
insoluble carrier particles of an insoluble carrier particle group with a large mean particle size support polyclonal antibodies and monoclonal antibodies, and
insoluble carrier particles of an insoluble carrier particle group with a small mean particle size support at least one of the polyclonal antibodies and the monoclonal antibodies.

4. The measuring reagent according to claim 1, wherein the insoluble carrier particles are latex particles.

5. The measuring reagent according to claim 2, wherein the antibody concentration ratio between the polyclonal antibodies and the monoclonal antibodies that are allowed to sensitize the insoluble carrier particles of the insoluble carrier particle group with the small mean particle size is 1:9 to 7:3.

6. A turbidimetric immunoassay in which, using a measuring reagent comprising an insoluble carrier particle group containing a plurality of insoluble carrier particles that support antibodies, an object to be measured to serve as antigens and the antibodies are reacted with each other, so that the insoluble carrier particle group is agglutinated,
wherein the measuring reagent to be used is a measuring reagent according to claim 1.

7. The turbidimetric immunoassay according to claim 6, wherein the insoluble carrier particle group in the measuring reagent comprises two types of insoluble carrier particle groups that are different in mean particle size from each other, and
in the two types of insoluble carrier particle groups,
insoluble carrier particles of an insoluble carrier particle group with a small mean particle size support polyclonal antibodies and monoclonal antibodies, and
insoluble carrier particles of an insoluble carrier particle group with a large mean particle size support at least one of the polyclonal antibodies and the monoclonal antibodies.

8. The turbidimetric immunoassay according to claim 6, wherein the insoluble carrier particle group in the measuring reagent comprises two types of insoluble carrier particle groups that are different in mean particle size from each other, and
in the two types of insoluble carrier particle groups,
insoluble carrier particles of an insoluble carrier particle group with a large mean particle size support polyclonal antibodies and monoclonal antibodies, and
insoluble carrier particles of an insoluble carrier particle group with a small mean particle size support at least one of the polyclonal antibodies and the monoclonal antibodies.

9. The turbidimetric immunoassay according to claim 6, wherein the insoluble carrier particles in the measuring reagent are latex particles.

10. The turbidimetric immunoassay according to claim 7, wherein the antibody concentration ratio between the polyclonal antibodies and the monoclonal antibodies that are allowed to sensitize insoluble carrier particles of the insoluble carrier particle group with the small mean particle size is 1:9 to 7:3.

11. A sample analysis tool that comprises a substrate and a measuring reagent and that is used for a turbidimetric immunoassay, with the measuring reagent being disposed on the substrate,
wherein the measuring reagent is a measuring reagent according to claim 1.

12. The sample analysis tool according to claim 11, wherein the insoluble carrier particle group in the measuring reagent comprises two types of insoluble carrier particle groups that are different in mean particle size from each other, and
in the two types of insoluble carrier particle groups,
insoluble carrier particles of an insoluble carrier particle group with a small mean particle size support polyclonal antibodies and monoclonal antibodies, and
insoluble carrier particles of an insoluble carrier particle group with a large mean particle size support at least one of the polyclonal antibodies and the monoclonal antibodies.

13. The sample analysis tool according to claim 11, wherein the insoluble carrier particle group in the measuring reagent comprises two types of insoluble carrier particle groups that are different in mean particle size from each other, and
in the two types of insoluble carrier particle groups,
insoluble carrier particles of an insoluble carrier particle group with a large mean particle size support polyclonal antibodies and monoclonal antibodies, and
insoluble carrier particles of an insoluble carrier particle group with a small mean particle size support at least one of the polyclonal antibodies and the monoclonal antibodies.

14. The sample analysis tool according to claim 12, wherein the antibody concentration ratio between the polyclonal antibodies and the monoclonal antibodies that are allowed to sensitize the insoluble carrier particles of the insoluble carrier particle group with the small mean particle size is 1:9 to 7:3.

15. The sample analysis tool according to claim 11, wherein the sample analysis tool is a test piece, a cartridge, or a microchip.
